Europäisches Patentamt

European Patent Office

Office européen des brevets

(11) Publication number: **0 273 116**
**A2**

# EUROPEAN PATENT APPLICATION

(21) Application number: 87114513.2

(22) Date of filing: 05.10.87

(51) Int. Cl.⁴: **C07K 7/06** , C07K 7/08 ,
C07K 7/10 , C07K 15/14 ,
G01N 33/569 , G01N 33/571 ,
A61K 39/095 , A61K 39/40 ,
C12N 15/00 , A61K 37/02

The title of the invention has been amended (Guidelines for Examination in the EPO, A-III, 7.3).

(30) Priority: 09.10.86 EP 86113993

(43) Date of publication of application:
06.07.88 Bulletin 88/27

(84) Designated Contracting States:
AT BE CH DE ES FR GB GR IT LI LU NL SE

(71) Applicant: **Max-Planck-Gesellschaft zur Förderung der Wissenschaften e.V.
Bunsenstrasse 10
D-3400 Göttingen(DE)**

(72) Inventor: **Meyer, Thomas F.
Vöchtingstrasse 1
D-7400 Tübingen(DE)**
Inventor: **Stern, Anne
Karwendelstrasse 10
D-8122 Penzberg(DE)**

(74) Representative: **Vossius & Partner
Siebertstrasse 4 P.O. Box 86 07 67
D-8000 München 86(DE)**

(54) Gonococcal and meningococcal polypeptides, vaccines and diagnostics.

㉤ The subject matter of the invention is a polypeptide which includes an amino acid residue sequence constituted by at least 5 and up to about 80 amino acid residues, and which is capable of immunologically mimicking a conserved antigenic determinant site of a conococcal opacity protein (Protein II) and/or meningococcal class 5 protein.

The polypeptide of the invention can be used as a vaccine or diagnostic for the prevention of gonorrhea and/or meningitidis.

EP 0 273 116 A2

# A vaccine or diagnostic composition and a polypeptide useful therefor

This invention concerns a vaccine or diagnostic for the prevention of gonorrhea and/or meningitis, utilizing antigenic and immunogenic parts of a protein or utilizing antibodies raised by such immunogens.

Neisseria gonorrhoeae, a gram-negative coccus, is the causative agent of the human sexually transmitted disease gonorrhae. In men, the disease is usually symptomatic in 90% of the cases. Without treatment, males may suffer local extension of the infection to the prostate and accessory glands, which may be an important cause of male infertility. In contrast, in 50% of infected women, symptoms are absent or not severe. In 10-15% of the women, the infection extends to the fallopian tubes, causing Salpingitidis, ectopic pregnancy, infertility and pelvic inflammatory disease. In 1-3% of the cases of men or women, a systemic invasion by the organism can occur, causing arthritis, endocarditis and disseminated infection. But with adequate treatment, the morbidity is slight. (Davis et al., In: Microbiology; Harper International Edition, 1980).

Neisseria meningitidis is the major agent of bacterial meningitis. The organisms usually inhabit the human nasopharyngeal area without any symptoms, enhancing the immunity of the host. When individuals without adequate levels of immunity acquire the meningococcus, the resulting nasopharyngeal infection may lead to Bacteremia, ordinarily followed by acute meningitis. Despite treatment of meningitis infections, case fatality rates are about 10%, and neurological sequelae are common (Davis et al., loc. cit.).

Despite the widespread use of antibodies, diseases caused by N. gonorrhoeae and N. meningitidis continues to be a serious problem in both developing and industrialized countries, due to the rise of penicillinase-producing strains. Gonorrhea vaccines based on the use of whole cells have been employed a number of times, but were not efficient (Greenburg et al., "Preliminary Studies On the Development Of A Gonococcal Vaccine"; Bull. Wld. Org. 45, 531 ,1971). Meningitis vaccines consisting of capsular polyssaccharides seem to be more suitable for epidemic control than for treatment.

In order to develop a more efficient vaccine or diagnosticum based on purified components, several investigations have been concentrated on surface antigens of N. gonorrhoeae and N. meningitidis, including lipopolysaccharides (LPS), peptidoglycan, IgA1 protease, pili and outer membrane proteins. The international application WO85/04654 proposes immunogenic pilus peptides encompasing two conserved regions within the predominantly variable C-terminus. These defined antigenic determinant sites are thought to be useful to prepare peptide vaccines. Due to the complexity of immunological reactions there, however, continues to exist a considerable need for the determination and provision of further antigenic determinant sites to amplify the scope and specificity of the vaccines or diagnostics.

It is an object of the present invention to provide a polypeptide and the pharmaceutically acceptable salts thereof which is capable of being an antigen, or an immunogen, against a gonococcal and/or meningococcal infection and which amplifies the scope and specificity of vaccines or diagnostics.

It is a further object of the present invention to provide diagnostic compositions comprising a polypeptide as mentioned above which are suitable for the detection of antibodies in a sample such as blood-serum of a patient.

An additional object of the present invention is to provide a diagnostic composition suitable for the detection of a gonococcal opacity protein (Protein II) and/or a meningococcal class 5 protein.

Furthermore it is an object of the present invention to provide a vaccine composition suitable for the prevention of gonorrhea and/or meningitis.

Still further it is an object of the present invention to provide a genetically modified bacterial of viral system which comprises a live vaccine suitable for the prevention of gonorrhea and/or meningitis and which elicits at least one of the above mentioned polypeptides.

Finally it is an object of the present invention to provide a method of immunizing a patient in which the vaccine composition or the genetically modified bacterial or viral systems mentioned above are administered or delivered via a suitable route, respectively.

According to the present invention, the solution of the above technical problems essentially is achieved by providing new conserved regions of the opacity protein (Op protein), also referred to as Protein II. On the surface of intact gonococci Op proteins can be assigned to serological classes with little antigenic relatedness, although these proteins share common peptides reminiscent of the constant regions of the pilus protein (Swanson and Barrera, J. Exp. Med. 157, 1405-1420, 1983; for detailed description see: Stern et al., Cell 37, 447-456, 1984). Most of the constant portions, revealed for the gonococcal opacity proteins, could be recovered in the meningococcal class 5 proteins. Apart from the constant regions, the class 5 protein of N. meningitidis share some additional characteristics with the opacity proteins of N.gonorrhoeae: (a) location in the outer membrane, (b) temperature-modifiable mobility in SDS polyacrylamide gels, (c) inter-strain and intra-strain variability of amino acid composition restricted to distinct areas of the proteins (Frasch and Mocca, J. Bacteriol., 78, 1127-1134, 1978; Zollinger and Mandrell, Infect. Immun. 28, 451-458, 1980; Judd, Infect. Immun. 48, 452-457, 1985).

Investigations have revealed that the colonization of gonococci in the human mucosa, the initial step of the infection, appears to be dependent on the expression of the pilus and the opacity protein. Preliminary studies suggest that the mechanisms of attachment to the mucosa by N.gonorrhoeae and N.meningitidis are similar or possibly identical (McGee et al., Rev. Infect. Dis. 5, 708-714, 1983). To complete a vaccine composed of constant pilus-specific peptides and/or to develop a diagnostic suitable for the pathogenic species N. gonorrhoeae and N. meningitidis, it was looked for conserved antigenic determinants that can be used for the preparation of antigens and/or immunogens that correspond to these antigenic determinants and that enhance antibody production in the host.

The polypeptide of this invention is capable of immunologically mimicking a conserved antigenic site of the opacity protein in N.gonorrhoeae and/or the class 5 proteins in N.meningitidis. It comprises at least one peptide of at least 5 amino acid residues up to about 2000 amino acid residues, that defines an immunogenic and antigenic site of a gonococcal Op protein and/or meningococcal class 5 proteins. Such a polypeptide is represented by an amino acid sequence of at least 5 up to about 80 amino acid residues that can be repeated as a unit one or more times in the same polypeptide molecule, or more than one type of such repeating unit can be present in a single polypeptide molecule. For further explanation see definition of the term "polypeptide", below.

Such polypeptide can be made as a fusion protein constructed by genetic engineering techniques or it can be synthesized from individual amino acid residues.

The corresponding DNA sequences can be either obtained from natural sources or they can be obtained by DNA synthesis according to known methods.

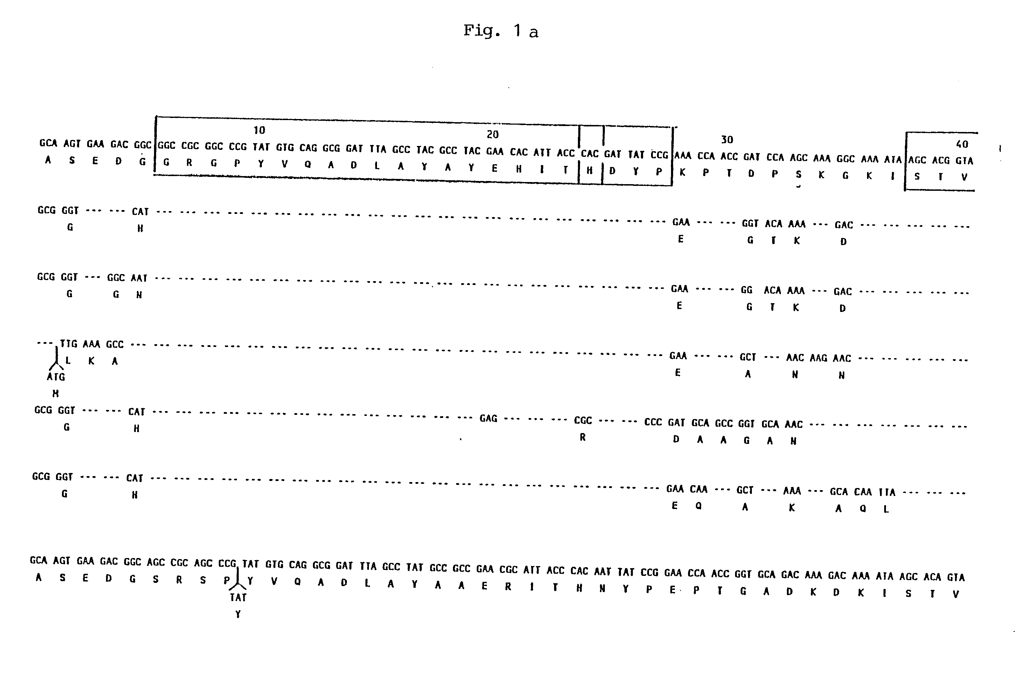

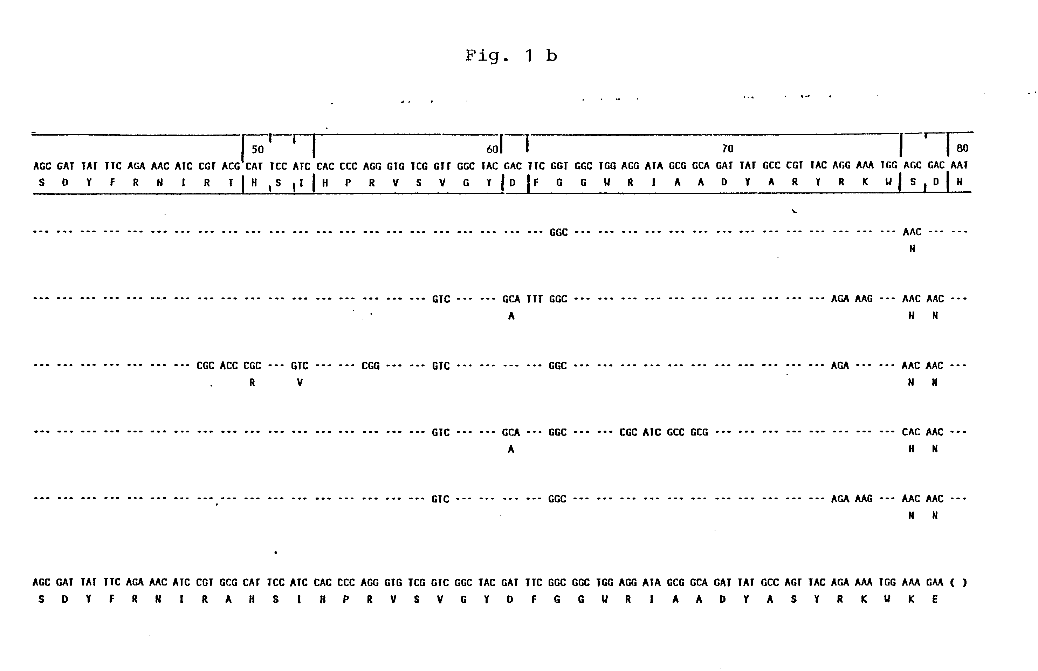

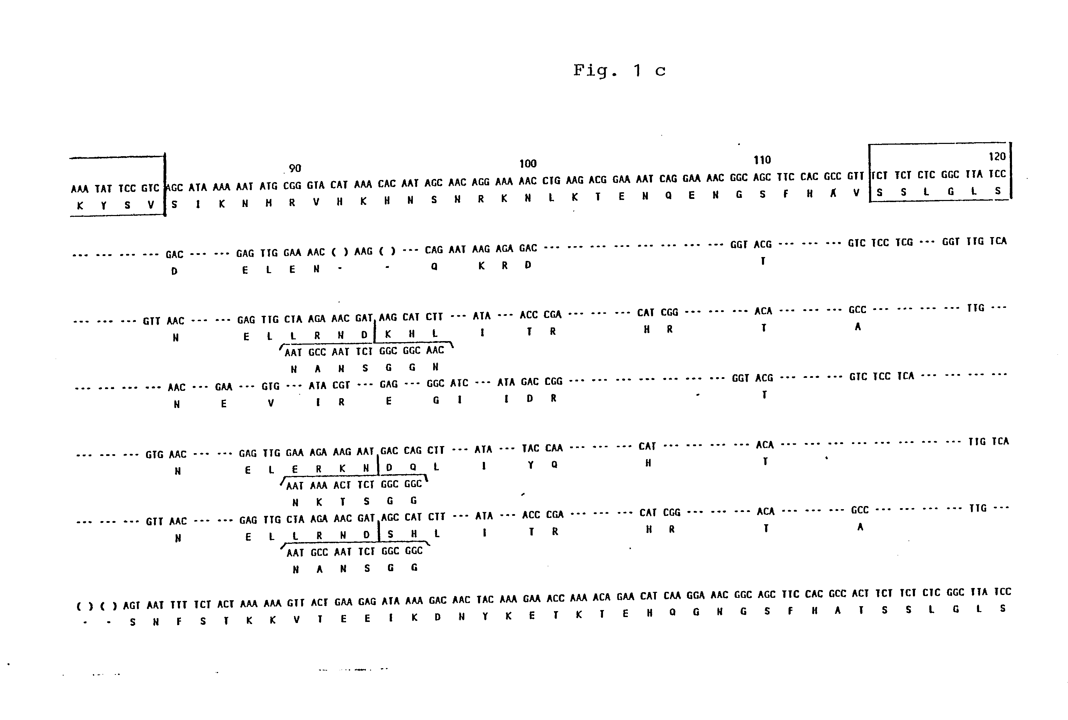

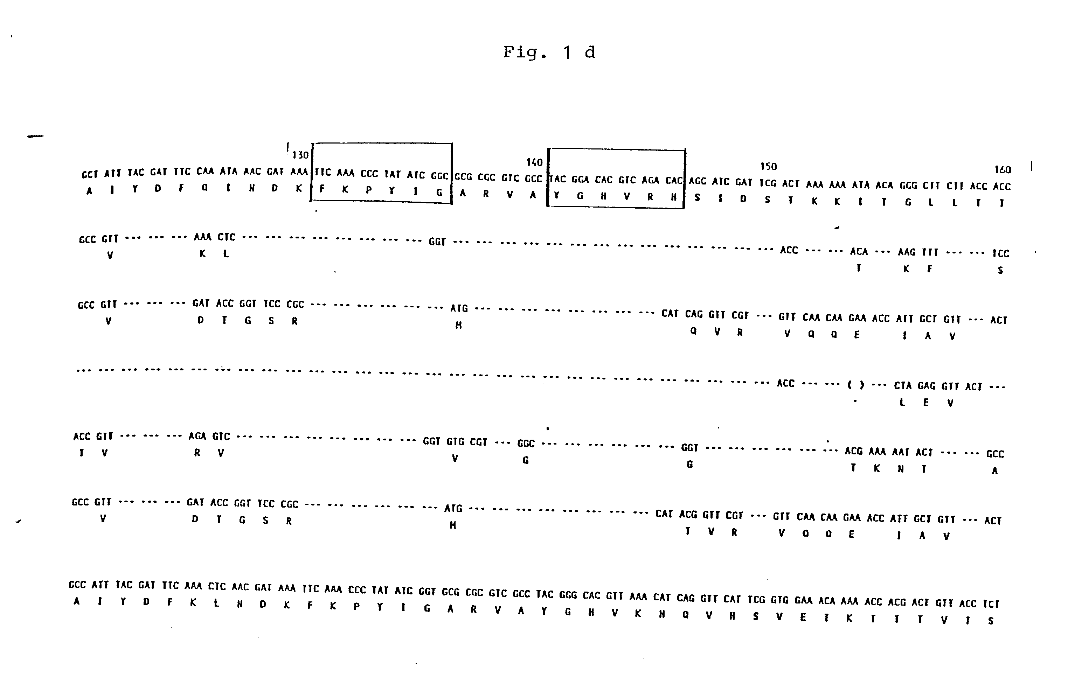

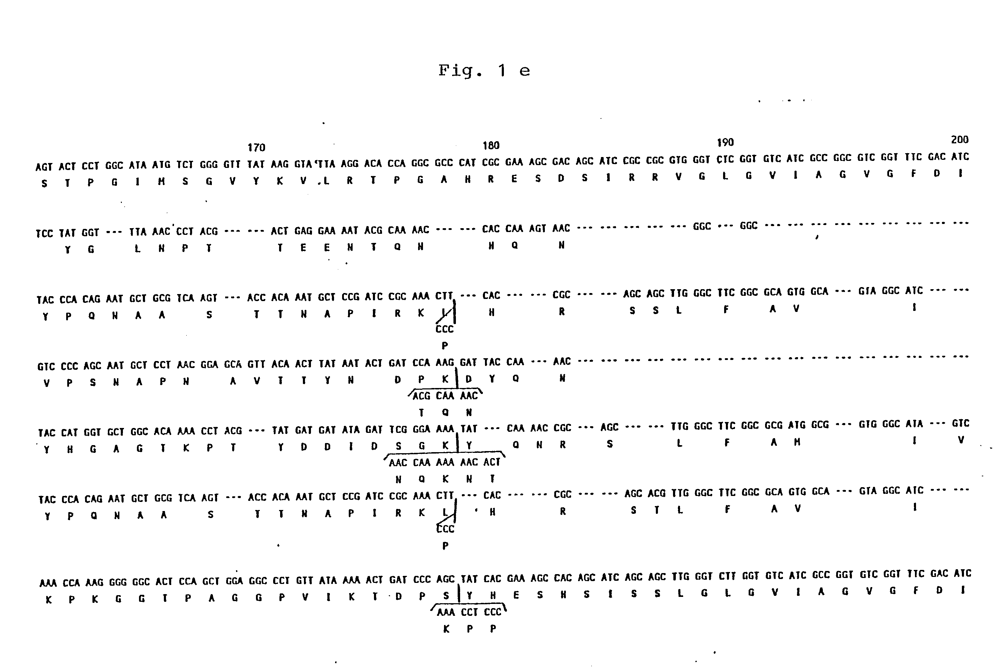

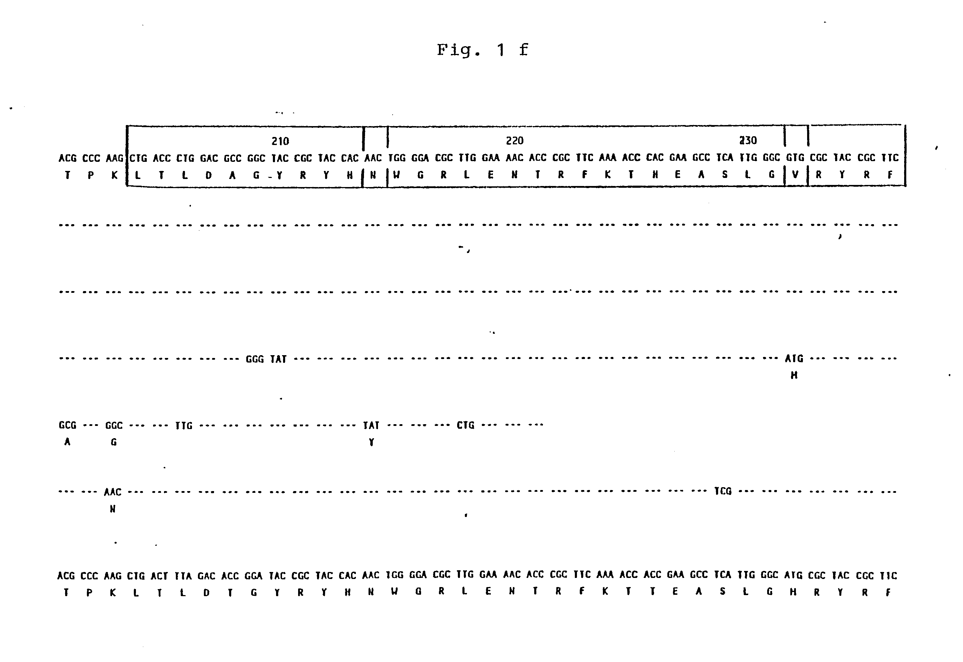

Polypeptides embodying this invention are deduced from the sequences of opacity proteins and/or class 5 proteins, summarized in Figure 1.

The term "polypeptide", as described herein, refers to a proteinous molecule that, as a whole, not typically occurs in nature, but results from intellectual and technical effort. Such a polypeptide preferentially consists of L-amino acid residues typically found in nature, however, chemical modifications of naturally-occuring amino acid residues may also be included in case they are capable of mimicking an antigenic determinant site. The polypeptides, as described herein, may be constituted by at least five amino acid residues and up to about 2000 amino acid residues. Typically such a polypeptide can be produced by chemical means (e.g., see Merrifield, J. Am. Chem. Soc. 85, 2149-2154, 1963) or by microorganisms which have been genetically engineered (e.g. see Silhavy et al., Microbiol. Rev. 47, 313-344, 1983). Polypeptides produced in that way should include at least one of the amino acid sequences described herein. The polypeptides also may include multiple identical sequences, and/or in conjunction with other amino acid sequences as described herein, and/or in conjunction with unrelated amino acid sequences.

"Amino acid sequences", as described herein, may represent at least five and up to about 80 amino acid residues of a continuous amino acid sequence of a gonococcal opacity protein and/or a meningococcal class 5 protein. The amino acid sequences, as described herein, should resemble at least one conserved antigenic determinant site of a gonococcal opacity protein (Protein II) and/or a meningococcal class 5 protein.

The term "antigenic determinant", as used herein, refers to a structural element of a polypeptide which is capable of interacting with a corresponding receptor molecule. A "conserved" antigenic determinant site, as described herein, is an amino acid sequence widely common to opacity proteins and meningococcal class 5 proteins, thus capable of binding to an identical receptor molecule, respectively. Figure 1 summarizes the DNA/protein sequences of 6 opacity proteins and I class 5 protein, providing a basis for this invention.

The phrase "immunologically mimicking" is used herein to mean that the polypeptides of this invention are not naturally occurring proteins or proteolytic products thereof. Furthermore, this phrase also includes the possibility that an "antigen related variant" of a given polypeptide is capable of binding to, and/or of eliciting the production of, an identical receptor molecule.

Multiple evidence for the existence of such antigenically related variants has been summarized recently; see WO85/04654, pp. 9-12. Data obtained from immuno assays and from studies of protein-protein interactions in solved X-ray crystallographic structures indicates that the protein-protein recognition does not require a strict conservation of the amino acid sequence for relatedness. A single amino acid residue exchange can influence the structure of a given polypeptide in different ways depending upon the nature of the substitution. The degree of relatedness of two different amino acid sequences can be expressed in terms of several amino acid parameters.

3

In this invention two polypeptides should be considered as two antigenically related variants provided that the confidence level of these amino acid parameters is not less than 95 %, or preferably, greater than 99 %.

The term "receptor molecule", as used in this invention, refers to a molecule to which a polypeptide, and an antigenically related variant thereof, carrying an antigenic determinant site can bind with high specificity. Such receptor molecules can be elicited by using a polypeptide as an immunogen in an animal host, preferentially a mammal. For this purpose the immunogen will be administered in a suitable form with respect of the carrier, the adjuvant, the dose, and the site of application, etc. The resulting immunoglobulines, i.e. the receptor molecules, as well as parts of the receptor molecules still capable of binding to the antigenic determinant site, i.e. their immunoglobuline combining sites, can be used for diagnostic purposes. Such receptor molecules might also be useful for passive protection against gonococcal infection.

A "diagnostic composition" includes one or more such receptor molecules specific for antigenic determinant sites in one or more polypeptides described in this invention. In addition, a diagnostic composition according to this invention includes the means, such as labelled secondary antibody, necessary to detect a specific reaction of a receptor molecule with its corresponding antigenic determinant site in a gonococcal opacity protein (protein II) and/or a meningococcal class 5 protein.

In humans the polypeptides described in this invention can be effective in preventing an infection with gonococci and/or meningococci. Two principal strategies can be chosen for an immunization against gonococcal and/or meningococcal infection using polypeptides described in this invention:

(a) A "vaccine composition" containing at least one polypeptide of this invention in combination with a suitable and pharmaceutically acceptable adjuvant and/or diluent. In case the polypeptide(s) used in this vaccine composition are not immunogenic by themselves, which is usually true for polypetides containing less than about 20 amino acid residues, the polypeptide is coupled to a suitable carrier molecule. A typical coupling reaction is described in Klipstein et al., J. Infect. Dis. 147, 318, 1983.

For immunizing a patient against gonorrhea and/or meningitis such a peptide vaccine composition will be administered in a suitable form by injection, orally or nasopharyngeally. The effective amounts of a polypeptide that is administered to a human by this method range from about 10 micrograms to about 100 milligrams without carrier.

(b) The peptides of this invention can be elicited by genetically modified living bacteria or by cells of a patient after infection with a genetically modified virus, thus constituting a live vaccine that can be used as an alternative method of immunizing a patient against gonorrhea and/or meningitis.

A genetically modified bacterial system suitable for this purpose typically consists of an attenuated bacterial host, in which expressable gene sequences have been incorporated capable of expressing (a) polypeptide(s) of this invention. The bacterial host modified in this way is capable of transiently (usually up to about 14 days) colonizing a mucosal site of the patient and, by delivering polypeptides of this invention, induces mucosal immunity against gonorrhea and/or meningitis. A typical bacterial host used for this purpose is Salmonella typhi Ty21a (Formal et al., Infect. Immun. 34, 746-750, 1981), but other suitable bacterial strains can be used too. Polypeptides of this invention are typically produced by the bacterial host as fusion proteins (Young et al., PNAS USA, 80, 1194-1198, 1984) that can be accumulated in, or released from, the cytoplasm of the bacterial host. The living bacteria are inocculated on mucosal surfaces of the patient in typical amounts of up to about $10^{12}$ colony forming units per individual. When administered orally the live bacterial vaccine is preferentially given in capsules in combination with neutralizing reagents.

A genetically modified viral system typically involves an attenuated vaccinia virus (see Brown et al., Nature 319, 549-550, 1986). For the purposes described in this invention, the genome of such a modified vaccinia virus contains the coding sequences for one or more polypeptides as described herein. Upon infection of the cells of a patient the viral vaccine gives rise to the production of polypeptides according to this invention. For immunizing a patient with a live viral vaccine of this invention up to about $10^{11}$ plaque-forming units are inocculated per individual, with preference intradermally or subcutaneously.

The Figures show:

Figure 1:

Comparison of the DNA and the amino acid sequences of 6 complete gonococcal opacity proteins (NG1-NG6) and 1 meningococcal class 5 protein (NM1).

The sequences were aligned using a computer program. All polypeptides corresponding to the invention are marked by frames.

Figure 2:

Distribution of hydrophilic and hydrophobic amino acid sequences within opacity and class 5 proteins exemplified by the hydropathy plot of the gonococcal protein NG2. In the plot the values for the leaderpeptide are included; therefore, the number of the amino acid positions do not correlate to the numbers used in Figure 1 and Table 1, respectively. Positive values refer to hydrophilic amino acid residues, negative values to hydrophobic amino acid residues. The locations of amino acid sequences selected for the synthesis of polypeptides are marked by arrows.

Example 1: Construction of Fusion Proteins Suitable for Diagnostic and Vaccine Purposes

Polypeptides of interest can be expressed as a portion of fusion proteins. For this purpose, defined segments of the cloned opacity gene including the coding regions for the conserved antigenic determinant sites are expressed in E.coli as fusion to the N-terminal part of the MS2-polymerase gene under the control of the inducible PL promoter (Strebel et al., J. Virol. 57, 983-991, 1986). But other expression systems yielding high amounts of fusion proteins can be used, too. First of all, it is necessary to enclose the opacity gene segments that encode the conserved antigenic determinant sites. Therefore, we constructed a series of Bal 31 derivatives of the opacity gene (NG1), truncated at the 5' end. After exonucleolytic digestion for different stretches, the truncated gene segments are subsequently fused to the MS2-polymerase coding part of the expression vector pEX. Thus the resulting fusion proteins harbour a 12 kd portion of the bacteriophage MS2-polymerase at their amino termini and opacity-specific sequences of different length at their carboxy termini. All constructs yield large amounts of proteins. Purification of fusion proteins is done as described by Strebel et al.(loc.cit.) A general explanation of the methods applied in carrying out the present invention can be found in Maniatis et al., "Molecular Cloning", CSH Laboratory, Cold Spring Harbor, New York (1982).

Example 2: Immunization

Rabbits can be utilized herein to raise anti-fusion protein antibodies. Therefore, the host animal is primed with about 150 µg of the purified fusion protein suspended in Freund's complete adjuvant. The rabbits are typically injected subcutaneously with the first inoculum. The rabbits are boosted at intervals of 10-14 days with the same amount of protein in Freund's incomplete adjuvant; therefore, the rabbits are injected intraperitoneally on day 10, 20 and 30, respectively, of the immunization schedule. Each immunization consists of four injections of the inoculum. Mice may be immunized in a similar way using about one tenths of the mentioned dose per injection.

Animals are typically bled 10 days after the last injection. Control pre-immune serum is obtained from each animal by bleeding just before the initial immunization.

Sera are titrated by enzyme-linked immunosorbent assay (Bittle et al., Nature 298, 30-33, 1983) with the purified antigen (used for immunization) as well as unpurified fusion proteins (total cell extract).

If desired, cross-reaction between the antisera due to the N-terminal MS2-specific sequences shared by all fusion proteins, could be eliminated by competing with an extract of E.coli cells expressing the MS2-polymerase fragment exclusively. Alternatively, control inoculum solutions can be prepared using the MS2-polymerase expressed by such E.coli strains.

Example 3: Western Blot Assay

In a Western blot assay the rabbit antisera are investigated for their ability to immunoreact with gonococcal opacity proteins and meningococcal class 5 proteins, respectively. For immunoblotting of these proteins from cell lysates, colonies are grown on agar plates and collected. Cells equivalent to 2 mg of total protein are suspended in 150 μl sample solution (Laemmli, Nature 227, 680-685, 1970) and homogenized by sonication. After boiling, an aliquot of 15 μl is loaded on a SDS-polyacrylamid gel and electrophoresed. The proteins are transferred from the gel onto nitrocellulose sheets at 1V/cm² (Blake et al., Anal. Biochem. 136, 175-179).

The rabbit anti-fusion protein antisera are diluted approximately 1:100 in 3% BSA/PBS. The nitrocellulose filter with the bound antigen is incubated in the diluted antisera overnight. The filters are washed repeatedly in 0,5% Tween/TBS, once in TBS, soaked in TBS/BSA for 5 min., and incubated in a solution containing protein A coupled to alkaline phosphatase (in TBS/BSA) at room temperature for 1 hour. The filters are washed as described previously and incubated in a 5-bromo-4-chloro-3-indoxylphosphate solution for about 15 min. at room temperature (for details see Blake et al., op. cit.).


Example 4: Selection of Amino Acid Sequences Used for the Synthesis of Polypeptides Suitable for Diagnostic and Vaccine Purposes

For the synthesis of polypeptides we select several amino acid sequences out of the opacity proteins and class 5 proteins corresponding the following criterions:

Criterion (1): the polypeptide sequence have to be in accordance with the claims of this invention.

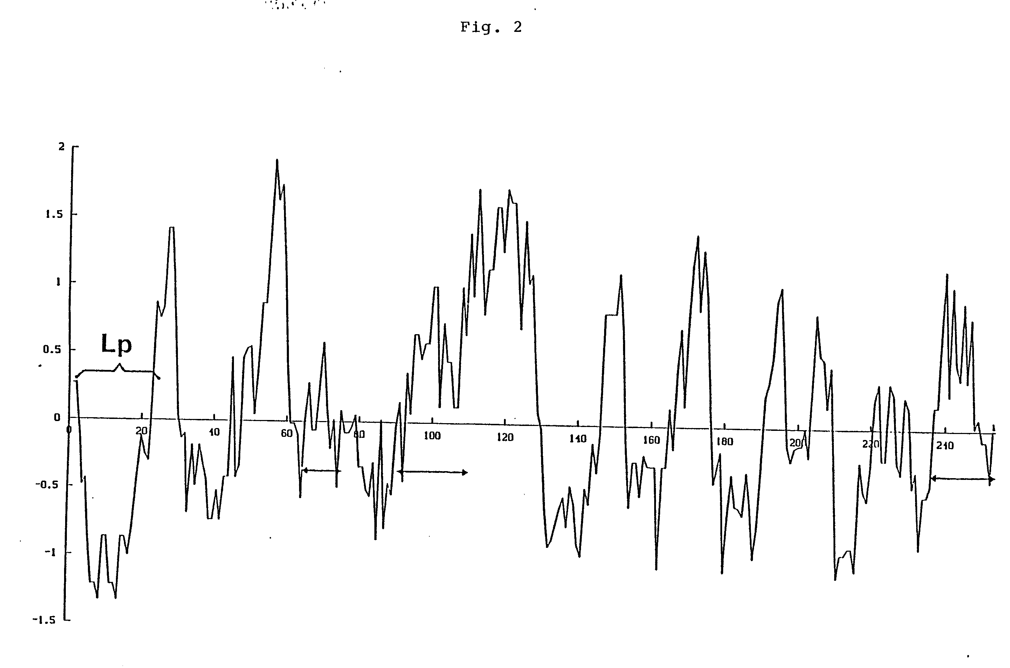

Criterion (2): the selected amino acid sequence should represent conserved antigenic determinant sites, which are mainly composed of hydrophilic amino acid residues (Fig. 2). Such conserved and hydrophilic amino acid sequences should be exposed on the surface of the gonococci and meningococci, respectively. Furthermore, such protein portions should fulfill general and important functions, for example as a binding site for receptors on mucosal cells. Such, probably immuno-recessive, epitopes of the opacity proteins and class 5 proteins should be rendered more immunogenic, when exposed as isolated polypeptides to the immune system of a patient.

Criterion (3): The DNA sequences coding for the selected polypeptides should be detectable in the genomes of all gonococcal and meningococcal strains and clinical isolates to ensure that the conservation criterion is not restricted to the strains investigated by DNA sequencing. A suitable test system therefor is a DNA dot blot assay using synthetic oligonucleotides as probes.

The polypeptide sequences selected by these criterions are shown in Table I .

Table I :  Polypeptides corresponding to conserved and hydrophilic regions of the opacity and class 5 proteins.

| POSITION | SEQUENCE (N-C) |
|---|---|
| 38-50 | STVSDYFRNIRTH |
| 66-84 | RIAADYARYRKWSDNKYSV* |
| 210-230 | YRYHNWGRLENTRFKTHEASL |

* not tested in the dot blot assay (Criterion 3)

**Claims**

1. A polypeptide which includes an amino acid sequence constituted by at least 5 and up to about 80 amino acid residues, and which is capable of immunologically mimicking a conserved antigenic determinant site of a gonococcal opacity protein (Protein II) and/or meningococcal class 5 protein.

2. The polypeptides in accordance with Claim 1 which include the amino acid sequences
GRGPYVQADLAYAYEHITHDYP or
GRGPYVQADLAYAYEHITRDYP or
parts of said amino acid sequences, as well as antigenically related variants of said polypeptides which are capable of immunologically mimicking a conserved antigenic determinant site of a gonococcal opacity protein (Protein II) and/or meningococcal class 5 protein.

3. The polypeptide in accordance with Claim 2 which includes the amino acid sequence
GRGPYVQADLAYAYEHIT or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

4. The polypeptides in accordance with Claim 1 which include the amino acid sequences
STVSDYFRNIRTHSIHPRVSVGYDFGGWRIAADYARYRKWSDNKYSV or
STVSDYFRNIRTHSIHPRVSVGYDFGGWRIAADYARYRKWNDNKYSV or
STVSDYFRNIRTHSIHPRVSVGYAFGGWRIAADYARYRKWNNNKYSV or
STVSDYFRNIRTRSVHPRVSVGYDFGGWRIAADYARYRKWNNNKYSV or
STVSDYFRNIRTHSIHPRVSVGYAFGGWRIAADYARYRKWHHNKYSV or
STVSDYFRNIRTHSIHPRVSVGYDFGGWRIAADYARYRKWNNNKYSV or
parts of said amino acid sequences, as well as antigenically related variants of said polypeptides which are capable of immunologically mimicking a conserved antigenic determinant site of a gonococcal opacity protein (Protein II) and/or meningococcal class 5 protein.

5. The polypeptide in accordance with claim 4 which includes the amino acid sequence
STVSDYFRNIRT or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

6. The polypeptide in accordance with Claim 4 which includes the amino acid sequence
HPRVSVGY or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

7. The polypeptide in accordance with Claim 4 which includes the amino acid sequence
FGGWRIAADYARYRKW or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

8. The polypeptide in accordance with Claim 4 which includes the amino acid sequence
NKYSV or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

9. The polypeptide in accordance with Claim 1 which includes the amino acid sequence
SSLGLS or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

10. The polypeptide in accordance with Claim 1 which includes the amino acid sequence
FKPYIG or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

11. The polypeptide in accordance with Claim 1 which includes the amino acid sequence
YGHVRH or
parts of said amino acid sequence, as well as antigenically related variants of said polypeptides which are capable of immunologically mimicking a conserved antigenic determinant site of a gonococcal opacity protein (Protein II) and or meningococcal class 5 protein.

12. The polypeptides in accordance with Claim 1 which include the amino acid sequences
LTLDAGYRYHNWGRLENTRFKTHEASLGVRYRF or
LTLDAGYRYHYWGRLENTRFKTHEASLGVRYRF or
LTLDAGYRYHNWGRLENTRFKTHEASLGMRYRF or
parts of said amino acid sequences, as well as antigenically related variants of said polypeptides which are capable of immunologically mimicking a conserved antigenic determinant site of a gonococcal opacity protein (Protein II) and/or meningococcal class 5 protein.

13. The polypeptide in accordance with Claim 12 which includes the amino acid sequence
LTLDAGYRYH or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

14. The polypeptide in accordance with Claim 12 which includes the amino acid sequence
WGRLENTRFKTHEASLG or
parts of said amino acid sequence, as well as antigenically related variants of said polypeptides which are capable of immunologically mimicking a conserved antigenic determinant site of a gonococcal opacity protein (Protein II) and/or meningococcal class 5 protein.

15. The polypeptides in accordance with Claims 2 to 14 having a proton at the amino terminus and a hydroxy group at the carboxy terminus, as well as a salt thereof.

16. A polypeptide containing up to about 2000 amino acid residues which consists of covalently linked multiple copies of at least one of the polypeptides according to anyone of claims 1 to 15 and optionally additionally of unrelated amino acid sequences.

17. A receptor molecule specifically binding to any of the polypeptides specified in Claims 1 to 16.

18. A receptor molecule raised in an animal host in accordance with Claim 17 wherein the receptor is a whole immunoglobuline.

19. A receptor molecule in accordance with Claim 17 wherein the receptor is an immunoglobuline combining site.

20. A diagnostic composition suitable for the detection of a gonococcal opacity protein (Protein II) and or a meningococcal class 5 protein, comprising
(a) a receptor molecule according to Claims 17 to 19 and
(b) means capable of signalling an immune reaction of said receptor molecules with a gonococcal opacity protein (Protein II) and/or a meningococcal class 5 protein.

21. A diagnostic composition suitable for the detection of antibodies directed to a gonococcal opacity protein (Protein II) and/or a meningococcal class 5 protein in a sample, comprising
(a) a polypeptide according to any one of claims 1 to 16 and
(b) means capable of signalling an immune reaction of said polypeptide with said antibodies contained in the sample to be tested.

22. A vaccine composition suitable for the prevention of gonorrhea and/or meningitis comprising at least one polypeptide of any of Claims 1 to 16.

23. A genetically modified bacterial or viral system, which comprises a live vaccine suitable for the prevention of gonorrhea and/or meningitis, and which elicits at least one polypeptide of any of Claims 1 to 16.

Fig. 1 a

```
                              10                              20            |  |         30                        40
GCA AGT GAA GAC GGC|GGC CGC GGC CCG TAT GTG CAG GCG GAT TTA GCC TAC GCC TAC GAA CAC ATT ACC|CAC GAT TAT CCG|AAA CCA ACC GAT CCA AGC AAA GGC AAA ATA|AGC ACG GTA
 A   S   E   D   G | G   R   G   P   Y   V   Q   A   D   L   A   Y   A   Y   E   H   I   T | H   D   Y   P | K   P   T   D   P   S   K   G   K   I | S   T   V

GCG GGT --- --- CAT --- --- --- --- --- --- --- --- --- --- --- --- --- --- --- --- --- --- --- --- --- --- --- GAA --- --- GGT ACA AAA --- GAC --- --- --- --- ---
 G           H                                                                                              E           G   T   K       D

GCG GGT --- GGC AAT --- --- --- --- --- --- --- --- --- --- --- ---, --- --- --- --- --- --- --- --- --- --- --- GAA --- --- GG  ACA AAA --- GAC --- --- --- --- ---
 G       G   N                                                                                              E           G   T   K       D

--- TTG AAA GCC --- --- --- --- --- --- --- --- --- --- --- --- --- --- --- --- --- --- --- --- --- --- --- --- GAA --- --- GCT --- AAC AAG AAC --- --- --- --- ---
 L   K   A                                                                                                  E           A       N   N
ATG
 M

GCG GGT --- --- CAT --- --- --- --- --- --- --- --- --- --- --- --- --- --- --- --- GAG --- --- --- CGC --- --- CCC GAT GCA GCC GGT GCA AAC --- --- --- --- --- ---
 G           H                                                                        R               D   A   A   G   A   N

GCG GGT --- --- CAT --- --- --- --- --- --- --- --- --- --- --- --- --- --- --- --- --- --- --- --- --- --- --- GAA CAA --- GCT --- AAA --- GCA CAA TTA --- --- ---
 G           H                                                                                              E   Q       A       K       A   Q   L


GCA AGT GAA GAC GGC AGC CGC AGC CCG TAT GTG CAG GCG GAT TTA GCC TAT GCC GCC GAA CGC ATT ACC CAC AAT TAT CCG GAA CCA ACC GGT GCA GAC AAA GAC AAA ATA AGC ACA GTA
 A   S   E   D   G   S   R   S   P   Y   V   Q   A   D   L   A   Y   A   A   E   R   I   T   H   N   Y   P   E   P   T   G   A   D   K   D   K   I   S   T   V
                         TAT
                          Y
```

## Fig. 1 b

AGC GAT TAT TTC AGA AAC ATC CGT ACG CAT TCC ATC CAC CCC AGG GTG TCG GTT GGC TAC GAC TTC GGT GGC TGG AGG ATA GCG GCA GAT TAT GCC CGT TAC AGG AAA TGG AGC GAC AAT
S   D   Y   F   R   N   I   R   T   H   S   I   H   P   R   V   S   V   G   Y   D   F   G   G   W   R   I   A   A   D   Y   A   R   Y   R   K   W   S   D   N

··· ··· ··· ··· ··· ··· ··· ··· ··· ··· ··· ··· ··· ··· ··· ··· ··· ··· ··· ··· ··· ··· ··· ··· GGC ··· ··· ··· ··· ··· ··· ··· ··· ··· ··· ··· ··· ··· ··· ··· ··· ··· ··· AAC ··· ··· ···
                                                                                                                                                            N

··· ··· ··· ··· ··· ··· ··· ··· ··· ··· ··· ··· ··· ··· ··· ··· ··· GTC ··· ··· GCA TTT GGC ··· ··· ··· ··· ··· ··· ··· ··· ··· ··· ··· ··· AGA AAG ··· AAC AAC ···
                                                                                A                                                          N       N

··· ··· ··· ··· ··· ··· ··· CGC ACC CGC ··· GTC ··· ··· CGG ··· ··· GTC ··· ··· ··· ··· GGC ··· ··· ··· ··· ··· ··· ··· ··· ··· ··· ··· AGA ··· ··· AAC AAC ···
                            R       V                                                                                                       N   N

··· ··· ··· ··· ··· ··· ··· ··· ··· ··· ··· ··· ··· ··· ··· ··· ··· GTC ··· ··· GCA ··· GGC ··· ··· CGC ATC GCC GCG ··· ··· ··· ··· ··· ··· ··· ··· ··· CAC AAC ···
                                                                                A                                                          H   N

··· ··· ··· ··· ··· ··· ··· ··· ··· ··· ··· ··· ··· ··· ··· ··· ··· GTC ··· ··· ··· ··· GGC ··· ··· ··· ··· ··· ··· ··· ··· ··· ··· ··· AGA AAG ··· AAC AAC ···
                                                                                                                                            N   N

AGC GAT TAT TTC AGA AAC ATC CGT GCG CAT TCC ATC CAC CCC AGG GTG TCG GTC GGC TAC GAT TTC GGC GGC TGG AGG ATA GCG GCA GAT TAT GCC AGT TAC AGA AAA TGG AAA GAA ( )
S   D   Y   F   R   N   I   R   A   H   S   I   H   P   R   V   S   V   G   Y   D   F   G   G   W   R   I   A   A   D   Y   A   S   Y   R   K   W   K   E

## Fig. 1 c

```
                              90                                    100                                   110                              120
AAA TAT TCC GTC AGC ATA AAA AAT ATG CGG GTA CAT AAA CAC AAT AGC AAC AGG AAA AAC CTG AAG ACG GAA AAT CAG GAA AAC GGC AGC TTC CAC GCC GTT TCT TCT CTC GGC TTA TCC
 K   Y   S   V  S   I   K   N   M   R   V   H   K   H   N   S   N   R   K   N   L   K   T   E   N   Q   E   N   G   S   F   H   K   V  S   S   L   G   L   S
```

```
--- --- --- --- GAC --- --- GAG TTG GAA AAC ( ) AAG ( ) --- CAG AAT AAG AGA GAC --- --- --- --- --- --- --- --- --- GGT ACG --- --- --- GTC TCC TCG --- GGT TTG TCA
                 D           E   L   E   N   -       -       Q   K   R   D                                           T
```

```
--- --- --- GTT AAC --- --- GAG TTG CTA AGA AAC GAT AAG CAT CTT --- ATA --- ACC CGA --- --- --- CAT CGG --- --- --- ACA --- --- --- GCC --- --- --- --- TTG ---
             N           E   L   L   R   N   D  K   H   L       I       T   R           H   R           T           A
                                             AAT GCC AAT TCT GGC GGC AAC
                                              N   A   N   S   G   G   N
--- --- --- --- AAC --- GAA --- GTG --- ATA CGT --- GAG --- GGC ATC --- ATA GAC CGG --- --- --- --- --- --- --- GGT ACG --- --- --- GTC TCC TCA --- --- --- ---
                 N       E       V       I   R       E       G   I       I   D   R                           T
```

```
--- --- --- GTG AAC --- --- GAG TTG GAA AGA AAG AAT GAC CAG CTT --- ATA --- TAC CAA --- --- --- CAT --- --- --- --- ACA --- --- --- --- --- --- --- --- TTG TCA
             N           E   L   E   R   K   N  D   Q   L       I       Y   Q           H                   T
                                             AAT AAA ACT TCT GGC GGC
                                              N   K   T   S   G   G
--- --- --- GTT AAC --- --- GAG TTG CTA AGA AAC GAT AGC CAT CTT --- ATA --- ACC CGA --- --- --- CAT CGG --- --- --- ACA --- --- --- GCC --- --- --- --- TTG ---
             N           E   L   L   R   N   D  S   H   L       I       T   R           H   R           T           A
                                             AAT GCC AAT TCT GGC GGC
                                              N   A   N   S   G   G
```

```
( ) ( ) AGT AAT TTT TCT ACT AAA AAA GTT ACT GAA GAG ATA AAA GAC AAC TAC AAA GAA ACC AAA ACA GAA CAT CAA GGA AAC GGC AGC TTC CAC GCC ACT TCT TCT CTC GGC TTA TCC
 -   -   S   N   F   S   T   K   K   V   T   E   E   I   K   D   N   Y   K   E   T   K   T   E   H   Q   G   N   G   S   F   H   A   T   S   S   L   G   L   S
```

Fig. 1 d

```
                                        130                140                150                    160
GCT ATT TAC GAT TTC CAA ATA AAC GAT AAA|TTC AAA CCC TAT ATC GGC|GCG CGC GTC GCC|TAC GGA CAC GTC AGA CAC|AGC ATC GAT TCG ACT AAA AAA ATA ACA GGG CTT CTT ACC ACC
 A   I   Y   D   F   Q   I   N   D   K | F   K   P   Y   I   G | A   R   V   A | Y   G   H   V   R   H | S   I   D   S   T   K   K   I   T   G   L   L   T   T


GCC GTT --- --- --- AAA CTC --- --- --- --- --- --- --- --- GGT --- --- --- --- --- --- --- --- --- --- --- --- --- ACC --- --- ACA --- AAG TTT --- --- TCC
 V           K   L                                                                                       T       K   F           S


GCC GTT --- --- --- GAT ACC GGT TCC CGC --- --- --- --- --- --- ATG --- --- --- --- --- --- --- --- --- CAT CAG GTT CGT --- GTT CAA CAA GAA ACC ATT GCT GTT --- ACT
 V           D   T   G   S   R                                H                       Q   V   R       V   Q   Q   E       I   A   V


... ... ... ... ... ... ... ... ... ... ... ... ... ... ... ... ... ... ... ... ... ... ... ... ... ... ... ACC --- --- ( ) --- CTA GAG GTT ACT ---
                                                                                                                         L   E   V


ACC GTT --- --- --- AGA GTC --- --- --- --- --- --- --- --- GGT GTG CGT --- GGC --- --- --- --- --- --- GGT --- --- --- --- --- --- ACG AAA AAT ACT --- --- GCC
 T   V       R   V                               V       G                       G                       T   K   N   T           A


GCC GTT --- --- --- GAT ACC GGT TCC CGC --- --- --- --- --- --- ATG --- --- --- --- --- --- --- --- --- CAT ACG GTT CGT --- GTT CAA CAA GAA ACC ATT GCT GTT --- ACT
 V           D   T   G   S   R                                H                       T   V   R       V   Q   Q   E       I   A   V


GCC ATT TAC GAT TTC AAA CTC AAC GAT AAA TTC AAA CCC TAT ATC GGT GCG CGC GTC GCC TAC GGG CAC GTT AAA CAT CAG GTT CAT TCG GTG GAA ACA AAA ACC ACG ACT GTT ACC TCT
 A   I   Y   D   F   K   L   N   D   K   F   K   P   Y   I   G   A   R   V   A   Y   G   H   V   K   H   Q   V   H   S   V   E   T   K   T   T   T   V   T   S
```

## Fig. 1 e

```
                        170                              180                             190                           200
AGT ACT CCT GGC ATA ATG TCT GGG GTT TAT AAG GTA'TTA AGG ACA CCA GGC GCC CAT CGC GAA AGC GAC AGC ATC CGC CGC GTG GGT CTC GGT GTC ATC GCC GGC GTC GGT TTC GAC ATC
 S   T   P   G   I   M   S   G   V   Y   K   V .L  R   T   P   G   A   H   R   E   S   D   S   I   R   R   V   G   L   G   V   I   A   G   V   G   F   D   I


TCC TAT GGT --- TTA AAC CCT ACG --- --- ACT GAG GAA AAT ACG CAA AAC --- --- CAC CAA AGT AAC --- --- --- --- --- GGC --- GGC --- --- --- --- --- --- --- --- ---
 Y   G       L   N   P   T           T   E   E   N   T   Q   N           H   Q   N


TAC CCA CAG AAT GCT GCG TCA AGT --- ACC ACA AAT GCT CCG ATC CGC AAA CTT --- CAC --- --- CGC --- --- AGC AGC TTG GGC TTC GGC GCA GTG GCA --- GTA GGC ATC --- ---
 Y   P   Q   N   A   A   S   T   T   N   A   P   I   R   K   L|      H       R       S   S   L       F       A   V                   I
                                                            CCC
                                                             P
GTC CCC AGC AAT GCT CCT AAC GGA GCA GTT ACA ACT TAT AAT ACT GAT CCA AAG GAT TAC CAA --- AAC --- --- --- --- --- --- --- --- --- --- --- --- --- --- --- --- ---
 V   P   S   N   A   P   N       A   V   T   T   Y   N       D   P   K|D   Y   Q       N
                                                           /ACG CAA AAC\
                                                             T   Q   N
TAC CAT GGT GCT GGC ACA AAA CCT ACG --- TAT GAT GAT ATA GAT TCG GGA AAA TAT --- CAA AAC CGC --- AGC --- --- TTG GGC TTC GGC GCG ATG GCG --- GTG GGC ATA --- GTC
 Y   H   G   A   G   T   K   P   T       Y   D   D   I   D   S   G   K|Y       Q   N   R       S           L       F       A   M                   I       V
                                                          /AAC CAA AAA AAC ACT\
                                                            N   Q   K   N   T
TAC CCA CAG AAT GCT GCG TCA AGT --- ACC ACA AAT GCT CCG ATC CGC AAA CTT --- CAC --- --- CGC --- --- AGC ACG TTG GGC TTC GGC GCA GTG GCA --- GTA GGC ATC --- ---
 Y   P   Q   N   A   A   S   T   T   N   A   P   I   R   K   L|  ' H       R       S   T   L       F       A   V                   I
                                                            CCC
                                                             P
AAA CCA AAG GGG GGC ACT CCA GCT GGA GGC CCT GTT ATA AAA ACT GAT CCC AGC TAT CAC GAA AGC CAC AGC ATC AGC AGC TTG GGT CTT GGT GTC ATC GCC GGT GTC GGT TTC GAC ATC
 K   P   K   G   G   T   P   A   G   G   P   V   I   K   T   D   P   S|Y   H   E   S   H   S   I   S   S   L   G   L   G   V   I   A   G   V   G   F   D   I
                                                             /AAA CCT CCC\
                                                                K   P   P
```

Fig. 1 f

```
                     210                    220                    230
ACG CCC AAG CTG ACC CTG GAC GCC GGC TAC CGC TAC CAC AAC TGG GGA CGC TTG GAA AAC ACC CGC TTC AAA ACC CAC GAA GCC TCA TTG GGC GTG CGC TAC CGC TTC
 T   P   K   L   T   L   D   A   G _ Y   R   Y   H   N   W   G   R   L   E   N   T   R   F   K   T   H   E   A   S   L   G   V   R   Y   R   F

--- --- --- --- --- --- --- --- --- --- --- --- --- --- --- --- --- --- --- --- --- --- --- --- --- --- --- --- --- --- --- --- --- --- --- --- ---

--- --- --- --- --- --- --- --- --- --- --- --- --- --- --- --- --- --- --- --- --- --- --- --- --- --- --- --- --- --- --- --- --- --- --- --- ---

--- --- --- --- --- GGG TAT --- --- --- --- --- --- --- --- --- --- --- --- --- --- --- --- --- --- --- --- --- --- --- --- ATG --- --- --- ---
                                                                                                                            H

GCG --- GGC --- --- TTG --- --- --- --- --- --- --- TAT --- --- --- CTG --- --- ---
 A       G                                           Y

--- --- AAC --- --- --- --- --- --- --- --- --- --- --- --- --- --- --- --- --- --- --- --- --- --- --- --- --- --- --- TCG --- --- --- --- --- ---
         N

ACG CCC AAG CTG ACT TTA GAC ACC GGA TAC CGC TAC CAC AAC TGG GGA CGC TTG GAA AAC ACC CGC TTC AAA ACC ACC GAA GCC TCA TTG GGC ATG CGC TAC CGC TTC
 T   P   K   L   T   L   D   T   G   Y   R   Y   H   N   W   G   R   L   E   N   T   R   F   K   T   T   E   A   S   L   G   H   R   Y   R   F
```

0 273 116

Fig. 2